Europäisches Patentamt

European Patent Office

Office européen des brevets

(11) Publication number: **0 365 048**
**A2**

## (12) EUROPEAN PATENT APPLICATION

(21) Application number: **89122016.2**

(22) Date of filing: **07.08.85**

(51) Int. Cl.⁵: **A61M 25/06**

(30) Priority: **07.08.84 JP 165452/84**

(43) Date of publication of application:
**25.04.90 Bulletin 90/17**

(60) Publication number of the earlier application in accordance with Art.76 EPC: **0 171 077**

(84) Designated Contracting States:
**BE DE FR GB IT SE**

(71) Applicant: **TERUMO KABUSHIKI KAISHA trading as TERUMO CORPORATION**
**44-1, 2-chome, Hatagaya Shibuya-Ku**
**Tokyo 151(JP)**

(72) Inventor: **Suzuki, Tatsuo**
**1-5-27-302, Sakuradai Oyamada**
**Machida-shi Tokyo(JP)**
Inventor: **Matsumoto, Atsushi Terumo**
**Kabushiki K.-Fujimi-Ryo**
**2517 Oomiya Fujinomiya-shi**
**Shizuoka-ken(JP)**

(74) Representative: **Henkel, Feiler, Hänzel & Partner**
**Möhlstrasse 37**
**D-8000 München 80(DE)**

(54) **Catheter introducer device.**

(57) The invention is directed to a catheter introducer device, comprising: a sheath portion (2) including a sheath (5) and a first hollow hub (4) fitted to a proximal end of said sheath (5) which is adapted to be inderted into blood vessels (18); a dilator (3) including a tube (7) detachably insertable into said sheath (5), and a second hub (6) fixed to a proximal end of said tube (7) which is adapted to guide a guide wire therethrough; first means for preventing relative axial movement of said sheath portion (2) and said dilator (3) when said tube (7) is inserted into said sheath (5) and second means for preventing relative circumferential rotation of said sheath portion (2) and said dilator (3) when said tube is inserted into said sheath (5), said first and second means being arranged betweeen said first and second hubs (4,5). In accordance with the invention said first and second means (9,10,11,12) are adapted to safely prevent relative axial movement and circumferential rotation of the sheath portion relative to the dilator.

F I G. 3

## Catheter introducer device

The present invention relates to a catheter introducer device defined by the precharacterizing features of the claim.

A catheter introducer device is known as a device for percutaneous insertion of a catheter into a blood vessel. Such a device is composed of a sheath portion having a sheath and a sheath hub, and a dilator portion having a tube and a dilator hub. When such a catheter introducer device is used, the dilator portion is set in the sheath portion and the dilator and sheath portions are inserted into a blood vessel via a guide wire.

The dilator and sheath portions can be easily inserted when they are simultaneously rotated during insertion. In a conventional catheter introducer device, no means for simultaneously rotating the sheath and dilator portions is included. In such a conventional catheter introducer device, in order to insert the assembly of the sheath and dilator portions into a blood vessel while preventing rotation between the sheath and dilator portions, the hubs in the two portions must either be clamped together, or the tube of the dilator portion must be firmly clamped at a position above the sheath of the sheath portion so as to simultaneously rotate the two portions, thus resulting in a complex procedure.

Yet, even if such complex operation is performed, only the sheath portion can be inserted, the dilator portion being impeded by resistance of tissue of the like.

A catheter introducer device of the kind defined at the preamble is known from the EP-A-64 212. The second hub of this known device is screwed on the first hollow hub. The threads of this screw, which are arranged between the first and second hubs, form first and second means for preventing the relative axial movement and the relative circumferntial rotation, if the second hub is completey screwed on the first hub. If, however, the second hub is not completely screwed into the first hub, axial movement as well as circumferential rotation therebetween and hence between the sheath portion and the dilator is not prevented. Further, the second hub needs to be tightly screwed into the first hub in order to prevent loosening of the screw connection. A loos fit of the two hubs, as aforementioned, however, is not suited to prevent axial movement and circumferential rotation.

The catheter introducer device known from the DE-A-2 305 640 comprises a male-female tapering which is intended to hold the two parts of this catheter introducer device together. This tapering constitutes means for preventing relative axial movement and circumferential rotation when tightly assembled. This male-female tapering has a circular cross section and hence does not provide a safe connection for the two parts of the catheter introducer device, which may be loosened upon manipulation of the device.

It is the object of the present invention to provide a catheter introducer device of the kind defined at the preamble with safe means for preventing relative axial movement and circumferential rotation of the sheath portion relative to the dilator.

This object is attained by the characterizing features of the claim.

This invention can be more fully understood from the following detailed description when taken in conjunction with the accompanying drawings, in which:

Fig. 1 is a side view of an assembled catheter introducer device of the present invention,

Fig. 2 is a partial sectional view of the main body of the device shown in Fig.1,

Fig. 3 is a perspective view of the fitting portions of of the device shown in Fig. 1. and

Fig.4 to 6 are diagrams for explaining the operation for inserting the device shown in the preceeding figures into a blood vessel.

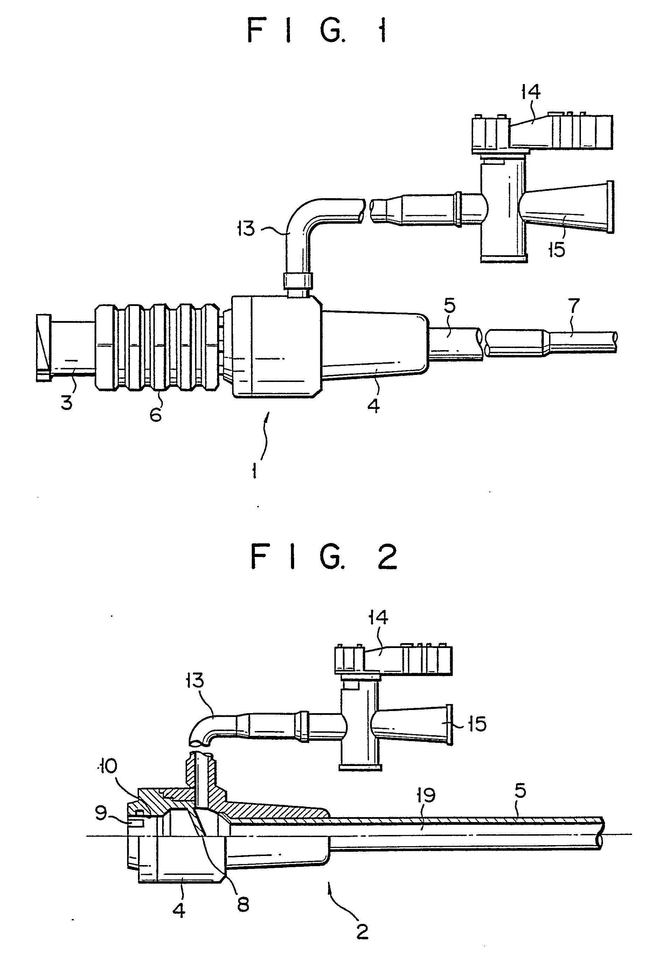

The catheter device 1 shown in Fig. 1 includes a main body 2 (Fig.2) and a rod member 3 The main body 2 corresponds to the sheath portion, and the rod member 3 corresponds to the dilator portion of a catheter introducer.

The main body 2 has a hub 4 and a sheath 5 having its proximal portion fixed and supported bY the hub 4, as shown in Fig.2 . The hub 4 and the sheath 5 have a through path 19 for receiving a tube 7 of the rod member 3.

A valve 8 is mounted, preferably, in the portion of the through path 19 in the hub 4. The valve 8 serves to prevent reverse flow of blood when the medical device main body is inserted into a blood vessel.

The main body 2 is connected at its hub portion 4 to a three port connection valve 14 through a side tube 13 communicating with the hub 4. This valve 14 can be operated to replenish a liquid through a lure taper port 15.

The rod member or dilator portion 3 has a hub 6 and a tube 7 fixed and supported thereby. The dilator portion 3 is inserted in and assembled with the main body 2 shown in Fig. 2. With this arrangement, when the main body 2 and the rod member 3 are percutaneously inserted into a blood vessel, they rotate relative to each other and, as such, may not lead themselves to easy insertion, as desribed above.

In view of this problem, according to the present invention a mechanism for preventing relative rotation between the main body 2 and the dilator portion 3 is incorporated.

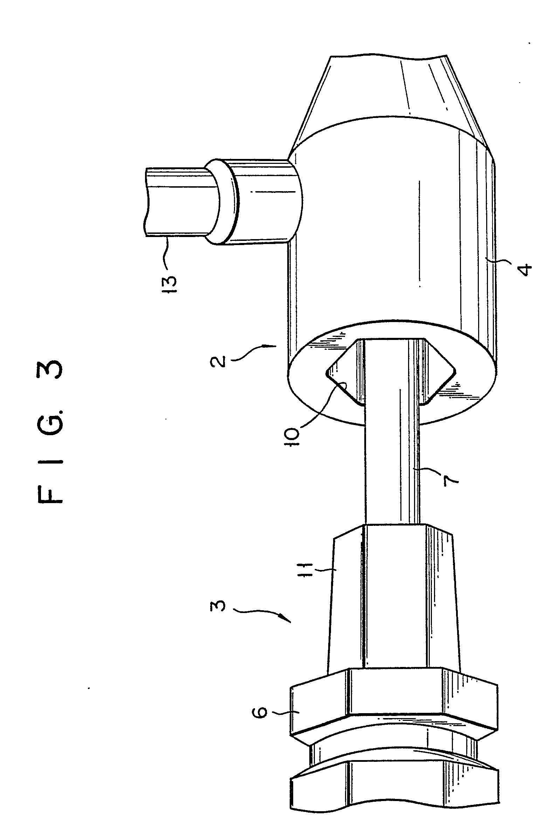

As shown in Fig. 3, said mechanism is provided between the main body 2 and the dilator portion 3 and comprises a male fitting portion 11 of the rod 3, and a female fitting portion 10 of the main body 2, said fitting portions being tapered and having hexagonal sections. Thus the fitting portions prevent axial displacement between the portions bY taper fitting, and also prevent rotation by hexagonal section fitting.

Fig. 1 shows a state wherein the sheath portion 2 in Fig. 2 is assembled with the dilator portion 3. Assembly can be performed as follows.

The tube 7 of the dilator portion 3 is inserted into the through path 19 in the hub 4 and the sheath 5 from the side of the hub 4 of the sheath portion 2. The hub 6 of the dilator portion 3 and the hub 4 of the sheath portion 3 are then fit together. At this time, the fitting portion 11 of the dilator portion 3 is fitted within the fitting portion 10 of the sheath portion 3 so as to prevent axial relative movement between the dilator and sheath portions 3 and 2.

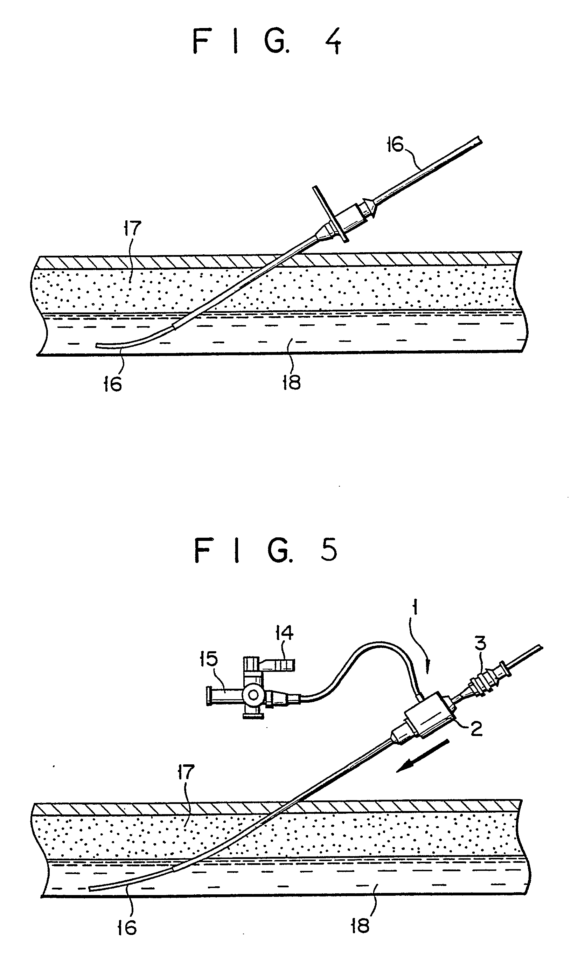

The method of using the device described above will be described with reference to Fig. 4 to 6.

A guide wire 16 is inserted into a blood vessel 18 through a subcutaneous tissue 17 by a suitable method such as the Seldinger method (Fig. 4). Then, the device main body 1, obtained by assembling the sheath portion (device main body) 2 shown in Fig. 2 and the dilator portion (rod member 3) so as to prevent axial movement and circumferential rotation as shown in Fig. 1, is inserted into a blood vessel 18 bY inserting the guide wire 16 into the tube 7, the sheath 5 and the through hole in hubs 4 and 6 (Fig. 5).

In this process, insertion into the blood vessel can be easily performed when the hub 6 of the dilator portion 3 is rotated during insertion.

In the catheter introducer device of the present invention, relative rotation between the dilator and sheath portions 3 and 2 is prevented by the hexagonal configuration of the surfaces of the fitting portions 10 and 11. In addition, axial movement between the portions 3 and 2 is prevented by a fitting force obtained bY the tapered configuration of the female and male fitting portions 10 and 11. Therefore, insertion of the device main body 1 into the blood vessel 18 through means of the guide wire 16 is facilitated.

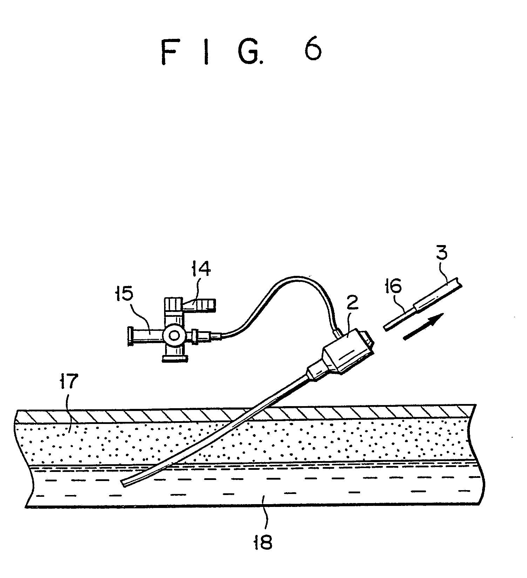

Subsequently, the sheath portion 6 is left in the blood vessel 18, and the guide wire 16 and the dilator portion 3 are pulled out (Fig. 6).

The catheter can be easily inserted into the blood vessel 18 through the indwelling sheath portion 2. Since the sheath portion 2 has a check valve 8, reverse flow of blood is prevented. If required, suction of a thrombus or injection of a physological saline solution can be performed through the lure taper port 15 of the sheath portion 2 before or after insertion of the catheter.

## Claims

1. A catheter introducer device, comprising:
a sheath portion (2) including a sheath (5) and a first hollow hub (4) fitted to a proximal end of said sheath (5) which is adapted to be inderted into blood vessels (18);
a dilator (3) including a tube (7) detachably insertable into said sheath (5), and a second hub (6) fixed to a proximal end of said tube (7) which is adapted to guide a guide wire therethrough;
first means for preventing relative axial movement of said sheath portion (2) and said dilator (3) when said tube (7) is inserted into said sheath (5) and second means for preventing relative circumferential rotation of said sheath portion (2) and said dilator (3) when said tube is inserted into said sheath (5),
said first and second means being arranged betweeen said first and second hubs (4,5),
characterized
in that said first means includes a male tapered surface (11) defining the outer surfac of said second hub (6) and a female tapered surface (10) defining the inner surface of said first hub (4), said male tapered surface (11) being fitted into said female tapered surface (10) to prevent said axial movement when said tube (7) is inserted into said first sheath (5), and in that said second means includes a female polyogonal prism surface (10) defining the inner surface of said first hub (4), and a male polygonal prism surface (11) defining the outer surface of said second hub (6), said male prism (11) being fitted into said female prism (10) to prevent rotation when said tube (7) is inserted into said sheath (5).

# F I G. 1

# F I G. 2

# F I G. 3

EP 0 365 048 A2

# F I G. 4

# F I G. 5

F I G. 6